# EUROPEAN PATENT SPECIFICATION

(11) **EP 1 767 219 B1**
(45) Date of publication and mention of the grant of the patent: **13.01.2010**
(21) Application number: 07075004.7
(22) Date of filing: 30.11.2004
(51) Int. Cl.: A61K 47/18, A61K 47/20, A61K 47/40, A61P 29/00

(54) **Stable injectable diclofenac compositions**
Stabile injizierbare Diclofenac- Zubereitungen
Préparations stables et injectables comprenant du diclofénac

(30) Priority: 10.03.2004 US 551351 P
(43) Date of publication of application: 28.03.2007
(62) Divisional of application: 04257437.6
(73) Proprietor: Shimoda Biotech (PTY) LTD, Port Elizabeth, Eastern Cape (ZA)
(72) Inventor: Penkler, Lawrence John, Port Elizabeth, Eastern Cape (ZA)
(74) Representative: Sayce, Alastair George

(56) References cited:
- EP-A- 0 658 347
- EP-A- 1 219 304
- WO-A-97/10805

## Description

### BACKGROUND TO THE INVENTION

Diclofenac is a leading non-steroidal anti-inflammatory drug (NSAID). The drug has been in clinical use for over two decades as a NSAID with analgesic, anti-inflammatory and anti-pyretic activity. Historically, diclofenac has been associated mainly with chronic management of inflammatory and degenerative forms of rheumatism as well as treatment of painful musculoskeletal conditions, acute attacks of gout, painful post-operative and post-traumatic inflammation and pain following dental surgery. For these conditions the drug has been available in delayed release enteric coated tablets, sustained release tablets, suppositories and ampoules for strict intramuscular injection. More recently, diclofenac has become available in rapid acting oral preparations for short term treatment of acute conditions. Since 1995, diclofenac sodium is available in the UK and Scandinavia as an intravenous infusion indicated for moderate to severe post-operative pain, or for the prophylaxis of post-operative pain.

Conventionally formulated diclofenac sodium injections are limited to intramuscular administration. This limitation has arisen, not as a consequence of the intravenous safety profile, but principally due to the physico-chemical properties of the drug, summarized as follows:
- Poor aqueous solubility of the sodium salt - Diclofenac has a particularly high tendency to crystallize from aqueous and organic solutions. Physically stable solutions containing at least 25 mg/ml of diclofenac sodium necessitates the use of potent solubilizing cosolvents, such as macrogols and benzyl alcohol. These cosolvents have an unfavourable intravenous safety profile and are associated with venous sequelae, high haemolytic and sensitising potential (see Reed, K.W. et al, J. Par. Sci. Technol. 39(2) (1985) 64-68).
- Susceptibility to oxidation - Diclofenac's tendency to oxidize in solution necessitates formulation with antioxidants, for example sulphite salts. In the commercial European intramuscular product, antioxidants such as sodium metabisulphite or sodium disulphite are usually used. Sulphite salts have been implicated in serious hypersensitivity reactions causing, for example, broncho-constriction (see Gunnison, A.F. et al, CRC Critical Reviews in Toxicology 17(3)(1987) 185-214).
- pH and Osmolality - The high pH of the marketed product (ca. 8.5) required to render diclofenac sodium soluble and the hyperosmolar nature of the formulation contribute to the discomfort which is frequently experienced at the site of the injection when administered intramuscularly.
- Injection Volume - Owing to poor solubility, the commercial product is formulated as 25mg diclofenac sodium per millilitre. The recommended dosage is 75mg and therefore the product is given as a 3 millilitre intramuscular injection. This is above the recommended volume of 2 millilitres for intramuscular injection accepted by the United States Food and Drug Administration.

US 5,679,660 to Farmarc Nederland BV teaches a method of preparing an injectable pharmaceutical or veterinary composition which comprises either diclofenac or a salt thereof and 2-hydroxypropyl beta-cyclodextrin with a preferred concentration of diclofenac of 25mg per millilitre. This reference discloses a method whereby the aqueous solubility of diclofenac was increased with the aid of a cyclodextrin to the extent that it could be formulated into a parenteral formulation containing 75mg diclofenac per 3ml. The formulatory volume of 3ml is not problematic with regard to the intravenous dosage route, as the drug will possibly be given by infusion, but as: far as the intramuscular dosage form is concerned, a volume of 3ml will not meet with FDA approval.

It has been found that solutions of diclofenac sodium in 2-hydroxypropyl beta-cyclodextrin prepared according to US 5,679,660 with a diclofenac sodium concentration of 25 mg per millilitre are stable for up to 12 months at room temperature and at least 24 months under refrigerated conditions. After 12 months at room temperature and 4 months at elevated temperature (e.g. 40 °C), appearance of visible insoluble particulate matter occurs which progresses with time. In order to satisfy a 24 month pharmaceutical shelf-life, the injectable product should be stored under refrigerated conditions.

A refrigerated parenteral product however has the disadvantage of discomfort upon injection due to the low temperature of the injected product coupled with the increased cost of product storage.

It is an object of this invention to provide a parenteral dosage form of diclofenac which addresses the aforementioned limitations and which may be used for both intramuscular and intravenous administration.

### SUMMARY OF INVENTION

According to the invention there is provided a stable aqueous solution as defined in claim 1.

The molar ratio of diclofenac to 2-hydroxypropyl beta-cyclodextrin is preferably 1:1:5 to 1:2.5, most preferably 1:2.

Typically, the solution comprises 20mg to 45mg, preferably more than 25mg, most preferably 37.5mg, diclofenac or diclofenac salt per millilitre solution.

The ethylene diamine tetra-acetic acid may comprise 0.05 to 1 mg, preferably 0.5 mg, per millilitre solution and the N-acetyl-cysteine may comprise 0.1 to 2 mg, preferably 1 mg, per millilitre solution.

Advantageously, the solution is in the form of a unit dose that does not exceed 2 millilitres.

### DETAILED DESCRIPTION OF THE INVENTION

The inventor has found a way to prepare an aqueous solution comprising either (a) diclofenac or a pharmaceutically acceptable diclofenac salt and a cyclodextrin, or (b) an inclusion complex of diclofenac or a pharmaceutically acceptable diclofenac salt and a cyclodextrin, or a mixture of (a) and (b), which is not only capable of having a concentration of diclofenac or diclofenac salt of more than 25 mg per millilitre of solution, but is also stable and does not need to be refrigerated when packed in clear glass pre-fillable syringes. By "stable" is meant that the solution can be stored for at least 12 months at room temperature and at least 6 months at elevated temperature (40°C) without the appearance of particulate matter which is visible to the eye.

The use of an antioxidant in the form of monothioglycerol (MTG) or a combination of ethylene diamine tetra-acetic acid (EDTA) and N-acetyl-cysteine (NAC) has been found to not only increase the diclofenac solubility to the extent that it is possible to dissolve 75mg of diclofenac-cyclodextrin into a final volume of 2ml (which means that the solubility of diclofenac (which is a very poorly water soluble drug) has been increased to such an extent that it could be formulated in a final volume 33% less than that proposed in US 5,679,660), but also effectively stabilises the solution preventing the formation of particulate matter at elevated temperature in pre-fillable syringes, ampoules and vials.

The solution may be formulated in unit dose form, each unit dose containing from 10 mg to 150 mg diclofenac or diclofenac salt inclusive, more preferably from 25mg to 75mg inclusive, most preferably 75 mg, in a volume not exceeding 2 millilitres.

The 2-hydroxypropyl beta-cyclodextrin (HPBCD) is selected from derivatives with a degree of substitution of between 2.5 and 10 hydroxypropyl substitutents per beta-cyclodextrin molecule, more preferably between 3.5 and 8 hydroxypropyl substitutents per beta-cyclodextrin molecule. The molar ratio of diclofenac to 2-hydroxypropyl beta-cyclodextrin is 1:1 to 1:10, more preferably 1:1.5 to 1:2.5, most preferably 1:2.

The injectable stabilised solution of the invention may be prepared by methods known in the art (e.g. US 5,679,660).

The stabilised injectable solution of the invention may be packed into suitable containers known in the art (for example glass ampoules, vials, cartridges, pre-filled syringes and the like). The glass should preferably be clear glass.

The stabilised injectable solution of the invention may be intravenously administered by admixture with non dextrose infusion fluids.

The stabilized injectable solution of the invention is suitable for intravenous and intramuscular use, saving money with regard to manufacturing cost and provides the patient with less discomfort due to a smaller intramuscular injection volume.

The stabilized injectable solution of the invention need not be stored under refrigerated conditions to provide a shelf life of at least 24 months, saving refrigeration costs during transport and storage, and alleviating patient discomfort during administration.

The antioxidants of the invention show advantages over a control solution containing no antioxidant and solutions containing other antioxidants, namely NAC or EDTA by themselves, sodium formaldehyde sulphoxilate (SFS) by itself and a combination of SFS and EDTA. Tables 1 and 2 below show stability evaluations of 75mg per 2ml diclofenac sodium formulations prepared according to the process disclosed in US 5,679,660 stored at 40°C for 3 and 6 months respectively. It is evident from Tables 1 and 2 that whereas the NAC EDTA combination according to the invention is stable after 6 months at 40°C, formulations containing NAC or EDTA by themselves, SFS by itself and a combination of SFS and EDTA are not stable.

**Table 1**

| Stability Evaluation Of 75mg/2ml Diclofenac Sodium Antioxidant Batches at 40°C For 3 Months | | | | |
|---|---|---|---|---|
| **Antioxidant** | **Chemical stability** | **pH trend at 40°C** | **Appearance/Particulate matter** | **Comments** |
| Control (No additives) | Acceptable level of known degradant | Upward | Discolouration; physical instability (+++) | Not stable (continued to 6 months as control) |
| EDTA 0.05% | Acceptable level of known degradant | Slightly upward | Discolouration; physical instability (+++) | Product not stabilised sufficiently-trial discontinued |
| NAC 0.1 % | Acceptable level of known degradant | Slightly upward | Stable | Complies, trial continued |
| NAC 0.1 % + EDTA 0.05% | Acceptable level of known degradant | Stable | Stable | Complies, trial continued |
| Monothioglycerol 0.1 % | Acceptable level of known degradant | Slightly upward | Stable | Complies, trial continued |
| Monothioglycerol 0.5% | Acceptable level of known degradant | Slightly upward | Stable | Complies, trial continued |
| Thioglycerol 0.1 % + EDTA 0.05% | Acceptable level of known degradant | Slightly upward | Stable | Complies, trial continued |
| SFS 0.005% | New degradant which exceeds 0.1% m/m | Upward | Physically unstable (+++) | Not stable-trial discontinued |
| SFS 0.1 % | New degradant which exceeds 0.1% m/m | Slightly upward | Discolouration; physically unstable, but more stable than 0.005% solution (++) | Not stable-trial discontinued |
| SFS 0.005% + EDTA 0.05% | Acceptable level of known degradant | Slightly upward | Physically unstable (++) | Not stable-trial discontinued |

| | | | | |
|---|---|---|---|---|
| Key: (+) = Very few colloidal particulates, fibres or filling artefacts (++) = Evidence of physical instability under light (+++) = Physical instability readily observable with the naked eye | | | | |

**Table 2**

| Stability Evaluation of 75mg/2ml Diclofenac Sodium AntiOxidant Batches at 40°C after 6 Months | | | | |
|---|---|---|---|---|
| **Antioxidant** | **Chemical stability** | **pH trend at 40°C** | **Appearance/Particulate matter** | **Comments** |
| Control (No additives) | Acceptable level of known degradant | Upward | Discolouration; physical instability (+++) | Not stable (Control) |
| NAC 0.1 % | Acceptable level of known degradant | Slightly upward | Discolouration; physical instability (+++) | Not stable |
| NAC 0.1 % + EDTA 0.05% | Acceptable level of known degradant | Slightly upward | Clear, straw colour - some particulate matter | Formulation possible |
| Monothioglycerol 0.1 % | Acceptable level of known degradant | Slightly upward | Clear, straw colour - some particulate matter (+) | Formulation possible |
| Monothioglycerol 0.5% | Acceptable level of known degradant | Slightly upward | Clear, straw colour - some particulate matter (+) | Formulation possible |
| Monothioglycerol 0.1 % + EDTA 0.05% | Acceptable level of known degradant | Slightly upward | Clear, straw colour - some particulate matter (+) | Formulation possible |

| | | | | |
|---|---|---|---|---|
| Key: (+) = Very few colloidal particulates, fibres or filling artefacts (++) = Evidence of physical instability under light (+++) = Physical instability readily observable with the naked eye | | | | |

The invention will now be described in more detail with reference to the following examples.

### EXAMPLE 1

The unit composition of a first preferred formulation of the invention is provided in Table 3 below:

**Table 3**

| **Ingredient** | **Quantity/2ml** |
|---|---|
| Diclofenac Sodium | 75 mg |
| Hydroxypropyl-β-cyclodextrin | 666 mg |
| N-acetyl-L-cysteine | 2 mg |
| Disodium edetate (EDTA) | 1 mg |
| Water for Injection to | 2 ml |
| Final pH | 6.5 - 8.5 |

### EXAMPLE 2 (This is not an example of the invention as claimed)

The unit composition of a second preferred formulation of the invention is provided in Table 4 below:

**Table 4**

| **Ingredient** | **Quantity/2ml** |
|---|---|
| Diclofenac Sodium | 75 mg |
| 2-Hydroxypropyl-β-cyclodextrin | 666 mg |
| Monothioglycerol | 10 mg |
| Water for Injection to | 2 ml |
| Final pH | 6.5 - 8.5 |

### EXAMPLE 3

Laboratory-scale formulations given in Examples 1 and 2 of the present invention were manufactured according to Example 4 of US 5,679,660 and filled into clear glass prefillable syringes and placed on a stability program. Table 5 below summarizes the results obtained:

**Table 5**

| STABILITY DATA FOR STABILIZED 75mg/2ml DICLOFENAC SODIUM-HPB SOLUTIONS | | | | | |
|---|---|---|---|---|---|
| Stabilised by: 0.5% m/v monothioglycerol | | | Stabilised by 0.05% m/v EDTA & 0.1 % m/v NAC | | |
| **T= 6 months** | | | **T=6 months** | | |
| | **25°C** | **40°C** | | **25°C** | **40°C** |
| **Appearance** | Clear colourless solution | Clear, straw coloured solution | **Appearance** | Clear, colourless solution | Clear, straw coloured solution |
| **PH** | 7.44 | 8.12 | **PH** | 7.17 | 8.16 |
| **Particulate Matter** | Free from visible particulate matter | Free from visible particulate matter | **Particulate Matter** | Free from visible particulate matter | Free from visible particulate matter |
| **Assay (HPLC) (% of T=0)** | ~100% | ~ 99% | **Assay (HPLC) (% of T=0)** | ~ 100% | ~ 99% |
| **Indolinone** | <0.1 % m/m | 0.57% | **Indolinone** | < 0.1 % | 0.53% m/m |
| **Other degradants** | None detected | None detected | Other degradants | None detected | None detected |

Control solutions at 40°C for 6 months showed heavy precipitation of an insoluble red-coloured material.

After 24 months at 25°C the solution containing monothioglycerol remained clear and slightly coloured, free from visible particulate matter. The associated solution containing N-acteyl-cysteine/EDTA was clear but more darkly colored than the monothioglycerol solution.

### EXAMPLE 4 This is not an example of the invention as claimed)

To produce 250 75mg/2ml diclofenac sodium units for IM or IV injection, 500ml water for injection (WFI) is purged with nitrogen gas to reduce the oxygen content to less than 0.5 mg/l. The water was heated to 50°C. Processing continues under a nitrogen gas blanket. 166.675g of HPBCD (DS 4.69) is added to 60% of the WFI batch volume and is mixed until dissolved. The solution is then allowed to cool to room temperature. The solution is pre-filtered with a 0.45µg filter, followed by the addition of 2.5 g MTG. The solution is stirred until all the MTG is dissolved. The pH is then adjusted to 4.5. 18.75 g diclofenac sodium is added to the solution and stirred until dissolved and made up to 100% volume with WFI and the pH is adjusted to 7.4, should it be required. The resultant 75mg/2ml diclofenac sodium solution is sterilized by filtration with 0.22µm filters and filled into pre-sterilized ampoules/vials under aseptic conditions. The ampoules/vials are sealed aseptically under nitrogen. The formulation contains 75.0±3.75 mg/2ml diclofenac sodium, as determined by validated HPLC.

### EXAMPLE 5

To produce 250 75mg/2ml diclofenac sodium units for IM or IV injection, 500ml water for injection (WFI) is purged with nitrogen gas to reduce the oxygen content to less than 0.5 mg/l. The water was heated to 50°C. Processing continues under a nitrogen gas blanket. 166.675g of HPBCD (DS 4.69) is added to 60% of the WFI batch volume and is mixed until dissolved. The solution is then allowed to cool to room temperature. The solution is pre-filtered with a 0.45µg filter, followed by the addition of 0.5 g NAC and 0.25 g EDTA. The solution is stirred until all the NAC and EDTA is dissolved. The pH is then adjusted to 4.5. 18.75 g diclofenac sodium is added to the solution and stirred until dissolved and made up to 100% volume with WFI and the pH is adjusted to 7.4, should it be required. The resultant 75mg/2ml diclofenac sodium solution is sterilized by filtration with 0.22µm filters and filled into pre-sterilized ampoules/vials under aseptic conditions. The ampoules/vials are sealed aseptically under nitrogen. The formulation contains 75.0±3.75 mg/2ml diclofenac sodium, as determined by validated HPLC.

### EXAMPLE 6

A production trial batch was produced according to the method as described in Example 5, whereby 15000 units of 75mg/2ml diclofenac sodium IM or IV units were produced. The stability of the formulations was monitored for 12 months at 25°C and 6 months at 40°C.

The stability trial results are summarized in Table 6 below:

**Table 6**

| SCREENING STABILITY TRIAL DATA | | | | | |
|---|---|---|---|---|---|
| Diclofenac Sodium 75mg/2ml (control) | | | Diclofenac Sodium 75mg/2ml | | |
| Stoichiometry ∼ 1:2 | | | (NAC/EDTA) | | |
| Control Batch (no stabilisers) | | | Stoichiometry ∼1:2 | | |
| Amber ampoule | | | Control Batch (0.05% EDTA + 0.1 % NAC) | | |
| | | | Amber ampoule | | |

| CONTROL BATCH | | | STABILISED BATCH | | |
|---|---|---|---|---|---|
| **T=3 months** | | | | | |
| | **25°C** | **40°C** | | **25°C** | **40°C** |
| Appearance | Clear colourless solution | Clear yellow solution | Appearance | Clear colourless solution | Clear, slightly straw colouried solution |
| pH | 7.25 | 7.74 | pH | 7.14 | 7.56 |
| Particulate Matter | Present (under direct light beam) | Present | Particulate Matter | Free from visible particulate matter | Free from visible particulate matter |
| Assay | 73.95 mg/2ml (99.8% of T=0) | 73.57 mg/2ml (99.3% of T=0) | Assay | 72.96 mg/2ml (99.9% of T=0) | 72.90 mg/2ml (99.8% of T=0) |
| Indolinone | <0.1 % | 0.23% m/m | Indolinone | nd | 0.31 % m/m |
| Other | nd | | Other | nd | nd |

| **T=6 months** | | | | | |
|---|---|---|---|---|---|
| | **25°C** | **40°C** | | **25°C** | **40°C** |
| Appearance | Straw coloured solution | Hazy, yellow - orange solution | Appearance | Clear colourless solution | Clear, slightly straw colourled solution |
| pH | 7.43 | 8.13 | pH | 7.25 | 8.47 |
| Particulate Matter | Present | Present | Particulate Matter | Free from visible particulate matter | Free from visible particulate matter |
| Assay | 74.81 mg/2ml (100.9%) | 74.28 mg/2ml (100.2%) | Assay | 73.47 mg/2ml (100.6%) | 72.88 mg/2ml (99.8%) |
| Indolinone | <0.1 % | 0.42% m/m | Indolinone | <0.1 % | 0.62% m/m |
| Other | nd | 0.11 % m/m | Other | nd | nd |

| **T=9 months** | | | | | |
|---|---|---|---|---|---|
| | **25°C** | **40°C** | | **25°C** | **40°C** |
| Appearance | Straw coloured solution | N/A | Appearance | Clear colourless solution | N/A |
| pH | 7.53 | N/A | pH. | 7.36 | N/A |
| Particulate Matter | Present | N/A | Particulate Matter | Free from visible particulate matter | N/A |
| Assay | 74.18 mg/2ml (100.1 %) | N/A | Assay | 73.32 mg/2ml (100.4%) | N/A |
| Indolinone | <0.1 % | N/A | Indolinone | 0.1 % | N/A |
| Other | nd | N/A | Other | nd | N/A |

| **T=12 months** | | | | | |
|---|---|---|---|---|---|
| | **25°C** | **40°C** | | **25°C** | **40°C** |
| Appearance | Straw coloured solution | N/A | Appearance | Clear colourless solution | N/A |
| pH | 7.62 | N/A | pH | 7.28 | N/A |
| Particulate Matter | Present | N/A | Particulate Matter | Free from visible particulate matter | N/A |
| Assay | 75.01 mg/2ml (101.2%) | N/A | Assay | 74.59 mg/2ml (102.1%) | N/A |
| Indolinone | <0.1 % m/m | N/A | Indolinone | 0.1 % m/m | N/A |
| Other | nd | N/A | Other | nd | N/A |

No physical or chemical instability was observed for the trial batches after 12 months at 25°C and 6 months at 40°C. There was no apparent difference between the upright or vertical orientation of the formulations.

## Claims

1. A stable aqueous solution comprising:
(a) diclofenac, a pharmaceutically acceptable diclofenac salt, an inclusion complex of diclofenac, or a combination thereof;
(b) a cyclodextrin; and
(c) an antioxidant which stabilises the solution, said antioxidant being a combination of ethylene diamine tetra-acetic acid and N-acetyl-cysteine.

2. A solution according to claim 1, wherein the ethylene diamine tetra-acetic acid is present in an amount from about 0.05 mg/ml to 1 mg/ml.

3. A solution according to claim 1, wherein the ethylene diamine tetra-acetic acid is present in an amount of about 0.5 mg/ml.

4. A solution according to claim 1, wherein the N-acetyl-cysteine is present in an amount from about 0.1 mg/ml to about 2 mg/ml.

5. A solution according to claim 1, wherein the N-acetyl-cysteine is present in an amount of about 1 mg/ml.

6. A solution according to claim 1, comprising:
| Ingredient | Quantity/2ml |
|---|---|
| Diclofenac Sodium | 75 mg |
| Hydroxypropyl-beta-cyclodextrin | 666 mg |
| N-acetyl-cysteine | 2 mg |
| Ethylene diamine tetra-acetic acid | 1 mg |
| Water for Injection to | 2 ml |
| Final pH | 6.5-8.5 |

7. A solution according to claim 1, wherein the diclofenac salt is diclofenac sodium.

8. A solution according to claim 1, wherein the cyclodextrin is 2-hydroxypropyl-beta-cyclodextrin.

9. A solution according to claim 8, wherein the molar ratio of diclofenac to 2-hydroxypropyl-beta-cyclodextrin is from about 1:1 to about 1:10.

10. A solution according to claim 8, wherein the molar ratio of diclofenac to 2-hydroxypropyl-beta-cyclodextrin is 1:1.5 to 1:2.5.

11. A solution according to claim 1, wherein the molar ratio of diclofenac to 2-hydroxypropyl beta-cyclodextrin is 1:2.

12. A solution according to claim 1, wherein the diclofenac, the pharmaceutically acceptable diclofenac salt, the inclusion complex of diclofenac, or the combination thereof is present in an amount of from about 20 mg/ml to about 45 mg/ml.

13. A solution according to claim 1, wherein the diclofenac, the pharmaceutically acceptable diclofenac salt, the inclusion complex of diclofenac, or the combination thereof is present in an amount of more than about 25mg/ml.

14. A solution according to claim 1, wherein the diclofenac, the pharmaceutically acceptable diclofenac salt, the inclusion complex of diclofenac, or the combination thereof is present in an amount of about 37.5 mg/ml.

15. A solution according to claim 1, in the form of a unit dose that does not exceed about 2 millilitres.

16. A solution according to claim 1, wherein the composition is administered intramuscularly.

17. A solution according to claim 1, wherein the composition is administered intravenously.

18. A solution according to claim 17, wherein the composition is administered intravenously by admixture with non-dextrose infusion fluids.

19. A solution according to claim 1, wherein the composition is stable when stored for more than 12 months at room temperature.

20. A solution according to claim 1, wherein the composition is stable when stored for more than 4 months at elevated temperatures.

21. A solution according to claim 1, wherein the composition is prepared in solution and stored in clear glass containers.

22. A solution according to claim 1, wherein the composition is stable when stored for more than 24 months at room temperature.

23. The use of a combination of ethylene diamine tetra-acetic acid and N-acetyl-cysteine to stabilise an aqueous solution comprising: diclofenac, a pharmaceutically acceptable diclofenac salt, an inclusion complex of diclofenac, or a combination thereof; and a cyclodextrin.

## Patentansprüche

1. Stabile wässrige Lösung, enthaltend:
(a) Diclofenac, ein pharmazeutisch zulässiges Diclofenac-Salz, ein Einschluss-Komplex von Diclofenac oder eine Kombination aus diesen;
(b) ein Cyclodextrin und
(c) ein Antioxidationsmittel, welches die Lösung stabilisiert, und wobei das Antioxidationsmittel eine Kombination von Ethylendiamintetraessigsäure und N-Acetylcystein ist.

2. Lösung nach Anspruch 1, wobei die Ethylendiamintetraessigsäure in einer Menge von ungefähr 0,05 mg/ml bis 1 mg/ml vorliegt.

3. Lösung nach Anspruch 1, wobei die Ethylendiamintetraessigsäure in einer Menge von ungefähr 0,5 mg/ml vorliegt.

4. Lösung nach Anspruch 1, wobei das N-Acetylcystein in einer Menge von ungefähr 0,1 mg/ml bis ungefähr 2 mg/ml vorliegt.

5. Lösung nach Anspruch 1, wobei das N-Acetylcystein in einer Menge von ungefähr 1 mg/ml vorliegt.

6. Lösung nach Anspruch 1, enthaltend:
| Bestandteil | Menge/2ml |
|---|---|
| Diclofenac-Natrium | 75 mg |
| Hydroxypropyl-Beta-Cyclodextrin | 666 mg |
| N-Acetylcystein | 2 mg |
| Ethylendiamintetraessigsäure | 1 mg |
| Wasser zur Injektion bis | 2 ml |
| Endgültiger pH-Wert | 6,5-8,5 |

7. Lösung nach Anspruch 1, wobei das Diclofenac-Salz Diclofenac-Natrium ist.

8. Lösung nach Anspruch 1, wobei das Cyclodextrin 2-Hydroxypropyl-Beta-Cyclodextrin ist.

9. Lösung nach Anspruch 8, wobei das molare Verhältnis von Diclofenac zu 2-Hydroxypropyl-Beta-Cyclodextrin ungefähr 1:1 bis ungefähr 1:10 beträgt.

10. Lösung nach Anspruch 8, wobei das molare Verhältnis von Diclofenac zu 2-Hydroxypropyl-Beta-Cyclodextrin 1:1,5 bis 1:2,5 beträgt.

11. Lösung nach Anspruch 1, wobei das molare Verhältnis von Diclofenac zu 2-Hydroxypropyl-Beta-Cyclodextrin 1:2 beträgt.

12. Lösung nach Anspruch 1, wobei das Diclofenac, das pharmazeutisch zulässige Diclofenac-Salz, der Einschluss-Komplex von Diclofenac oder die Kombination aus diesen in einer Menge von ungefähr 20 mg/ml bis ungefähr 45 mg/ml vorliegt.

13. Lösung nach Anspruch 1, wobei das Diclofenac, das pharmazeutisch zulässige Diclofenac-Salz, der Einschluss-Komplex von Diclofenac oder die Kombination aus diesen in einer Menge von mehr als ungefähr 25 mg/ml vorliegt.

14. Lösung nach Anspruch 1, wobei das Diclofenac, das pharmazeutisch zulässige Diclofenac-Salz, der Einschluss-Komplex von Diclofenac oder die Kombination aus diesen in einer Menge von ungefähr 37,5 mg/ml vorliegt.

15. Lösung nach Anspruch 1, in Form einer Einheitsdosis, welche nicht größer als ungefähr 2 Milliliter ist.

16. Lösung nach Anspruch 1, wobei die Zusammensetzung intramuskulär verabreicht wird.

17. Lösung nach Anspruch 1, wobei die Zusammensetzung intravenös verabreicht wird.

18. Lösung nach Anspruch 17, wobei die Zusammensetzung intravenös unter Zumischung von Infusionsflüssigkeiten ohne Dextrose verabreicht wird.

19. Lösung nach Anspruch 1, wobei die Zusammensetzung stabil ist, wenn sie für mehr als 12 Monate bei Raumtemperatur gelagert wird.

20. Lösung nach Anspruch 1, wobei die Zusammensetzung stabil ist, wenn sie für mehr als 4 Monate bei erhöhten Temperaturen gelagert wird.

21. Lösung nach Anspruch 1, wobei die Zusammensetzung in Lösung hergestellt und in Klarglasbehältem gelagert wird.

22. Lösung nach Anspruch 1, wobei die Zusammensetzung stabil ist, wenn sie für mehr als 24 Monate bei Raumtemperatur gelagert wird.

23. Benutzen einer Kombination von Ethylendiamintetraessigsäure und N-Acetylcystein zum Stabilisieren einer wässrigen Lösung, enthaltend: Diclofenac, ein pharmazeutisch zulässige Diclofenac-Salz, ein Einschluss-Komplex von Diclofenac oder eine Kombination von diesen und ein Cyclodextrin.

## Revendications

1. Solution aqueuse stable comprenant :
(a) du diclofénac, un sel de diclofénac pharmaceutiquement acceptable, un complexe d'inclusion de diclofénac, ou une combinaison de ceux-ci ;
(b) une cyclodextrine ; et
(c) un antioxydant qui stabilise la solution, ledit antioxydant étant une combinaison d'acide éthylène-diamine tétra-acétique et de N-acétyl-cystéine.

2. Solution selon la revendication 1, dans laquelle l'acide éthylène-diamine tétra-acétique est présent en une quantité allant d'environ 0,05 mg/ml à 1 mg/ml.

3. Solution selon la revendication 1, dans laquelle l'acide éthylène-diamine tétra-acétique est présent en une quantité d'environ 0,5 mg/ml.

4. Solution selon la revendication 1, dans laquelle la N-acétyl-cystéine est présente en une quantité allant d'environ 0,1 mg/ml à environ 2 mg/ml.

5. Solution selon la revendication 1, dans laquelle la N-acétyl-cystéine est présente en une quantité d'environ 1 mg/ml.

6. Solution selon la revendication 1, comprenant :
| Ingrédient | Quantité/2 ml |
|---|---|
| Diclofénac sodique | 75 mg |
| Hydroxypropyl-béta-cyclodextrine | 666 mg |
| N-acétyl-cystéine | 2 mg |
| Acide éthylène-diamine tétra-acétique | 1 mg |
| Eau à injecter jusqu'à | 2 ml |
| pH final | 6,5 - 8,5 |

7. Solution selon la revendication 1, dans laquelle le sel de diclofénac est du diclofénac sodique.

8. Solution selon la revendication 1, dans laquelle la cyclodextrine est la 2-hydroxypropyl-béta-cyclodextrine.

9. Solution selon la revendication 8, dans laquelle le rapport molaire du diclofénac à la 2-hydroxypropyl-béta-cyclodextrine est dans la plage allant d'environ 1 : 1 à environ 1 : 10.

10. Solution selon la revendication 8, dans laquelle le rapport molaire du diclofénac à la 2-hydroxypropyl-béta-cyclodextrine est dans la plage allant de 1 : 1,5 à 1 : 2,5.

11. Solution selon la revendication 1, dans laquelle le rapport molaire du diclofénac à la 2-hydroxypropyl-béta-cyclodextrine est de 1 : 2.

12. Solution selon la revendication 1, dans laquelle le diclofénac, le sel de diclofénac pharmaceutiquement acceptable, le complexe d'inclusion de diclofénac, ou la combinaison de ceux-ci est présent(e) en une quantité allant d'environ 20 mg/ml à environ 45 mg/ml.

13. Solution selon la revendication 1, dans laquelle le diclofénac, le sel de diclofénac pharmaceutiquement acceptable, le complexe d'inclusion de diclofénac, ou la combinaison de ceux-ci est présent(e) en une quantité supérieure à environ 25 mg/ml.

14. Solution selon la revendication 1, dans laquelle le diclofénac, le sel de diclofénac pharmaceutiquement acceptable, le complexe d'inclusion de diclofénac, ou la combinaison de ceux-ci est présent(e) en une quantité d'environ 37,5 mg/ml.

15. Solution selon la revendication 1, sous la forme d'une dose unitaire qui n'excède pas environ 2 millilitres.

16. Solution selon la revendication 1, dans laquelle la composition est administrée de manière intramusculaire.

17. Solution selon la revendication 1, dans laquelle la composition est administrée de manière intraveineuse.

18. Solution selon la revendication 17, dans laquelle la composition est administrée de manière intraveineuse après mélange avec des fluides de perfusion sans dextrose.

19. Solution selon la revendication 1, dans laquelle la composition est stable lorsqu'elle est stockée pendant plus de 12 mois à température ambiante.

20. Solution selon la revendication 1, dans laquelle la composition est stable lorsqu'elle est stockée pendant plus de 4 mois à des températures élevées.

21. Solution selon la revendication 1, dans laquelle la composition est préparée sous forme de solution et stockée dans des récipients en verre transparent.

22. Solution selon la revendication 1, dans laquelle la composition est stable lorsqu'elle est stockée pendant plus de 24 mois à température ambiante.

23. Utilisation d'une combinaison d'acide éthylène-diamine tétra-acétique et de N-acétyl-cystéine pour stabiliser une solution aqueuse comprenant : du diclofénac, un sel de diclofénac pharmaceutiquement acceptable, un complexe d'inclusion de diclofénac, ou une combinaison de ceux-ci ; et une cyclodextrine.
